# EUROPÄISCHE PATENTSCHRIFT

(11) **EP 1 131 061 B1**
(45) Veröffentlichungstag und Bekanntmachung des Hinweises auf die Patenterteilung: **01.06.2005**
(21) Anmeldenummer: 99972533.6
(22) Anmeldetag: 05.11.1999
(51) Int. Cl.: A61K 31/00, A61K 31/045, A61K 31/22, A61K 31/34, A61P 5/08

(54) **PROLAKTIN-SENKENDES MITTEL**
AGENT FOR LOWERING PROLACTIN
AGENT ABAISSANT LA TENEUR EN PROLACTINE

(30) Priorität: 19.11.1998 DE 19853476
(43) Veröffentlichungstag der Anmeldung: 12.09.2001
(73) Patentinhaber: Bionorica AG, 92318 Neumarkt (DE)
(72) Erfinder: WUTTKE, Wolfgang, D-37120 Bovenden (DE); JARRY, Hubertus, D-37249 Neu-Eichenberg (DE); POPP, Michael, D-91207 Lauf/Pegnitz (DE); CHRISTOFFEL, Volker, D-92369 Sengenthal (DE); SPENGLER, Barbara, D-92318 Neumarkt/Opf. (DE)
(74) Vertreter: Kaiser, Jürgen, Dr.rer.nat.Dipl.-Chem.
(86) Internationale Anmeldenummer: PCT/EP1999/008507
(87) Internationale Veröffentlichungsnummer: WO 2000/030623

(56) Entgegenhaltungen:
- WO-A-96/24684
- WO-A-99/63978
- SLIUTZ G; SPEISER P; SCHULTZ A M; SPONA J; ZEILLINGER R: "Agnus castus extracts inhibit prolactin secretion of rat pituitary cells" HORMONE AND METABOLIC RESEARCH , Bd. 25, 1993, Seiten 253-255, XP000913717
- MILEWICZ A; GEJDEL E; SWOREN H; SIENKIEWICZ K; JEDRZEJAK J; TEUCHER T; SCHMITZ H: "Vitex agnus castus extract in the treatment of luteal phase defects due to latent hyperprolactinaemia: Results of a randomized placebo-controlled double blind study." ARZNEIMITTEL-FORSCHUNG, Bd. 43, 1993, Seiten 752-756, XP000913700
- JARRY H; LEONHARDT SABINE; GORKOW C; WUTTKE W: "In vitro prolactin but not LH and FSH release is inhibited by compounds in extracts of Agnus castus: Direct evidence for a dopaminergic principle by the dopamine receptor assay." EXPERIMENTAL AND CLINICAL ENDOCRINOLOGY, Bd. 102, 1994, Seiten 448-454, XP000913581
- HAN, BYUNG HOON ET AL: "In Vitro Platelet-Activating Factor Receptor Binding Inhibitory Activity of Pinusolide Derivatives: A Structure-Activity Study" J. MED. CHEM. (1998), 41(14), 2626-2630 , 1998, XP000907424
- CAHILL D J; FOX R; WARDLE P G; HARLOW C R: "Multiple follicular development associated with herbal medicine." HUMAN REPRODUCTION (OXFORD), Bd. 9, 1994, Seiten 1469-1470, XP000913660
- TAGUCHI, HEIHACHIRO: "Studies on the constituents of Vitex cannabifolia" CHEM. PHARM. BULL. (1976), 24(7), 1668-70 , XP000907422
- KONDO Y ET AL: "STUDIES ON THE CONSTITUENTS OF VITEX-ROTUNDIFOLIA L. FIL." CHEM PHARM BULL (TOKYO), (1986) 34 (11), 4829-4932. , XP000907421
- MALES Z; BLAZEVIC N: "Composition of the essential oil of Vitex agunus-castus L. f. rosea fruits." PHARMAZIE, Bd. 53, 1998, Seiten 728-729, XP000907423
- OHSAKI, AYUMI ET AL: "The isolation and in vivo potent antitumor activity of clerodane diterpenoid from the oleoresin of the Brazilian medicinal plant, Copaifera langsdorfii Desfon" BIOORG. MED. CHEM. LETT. (1994), 4(24), 2889-92 ,1994, XP000913600
- CHEN, ZHENG-PING ET AL: "Studies on the antitumor, antibacterial, and wound-healing properties of dragon's blood" PLANTA MED. (1994), 60(6), 541-5 ,1994, Seiten 1469-1470, XP000913598
- ROJAS, A. ET AL: "Smooth muscle-relaxing compounds from Dodonaea viscosa" PLANTA MED. , Bd. 62, Nr. 2, 1996, Seiten 154-159, XP000913597

## Beschreibung

Die vorliegende Erfindung betrifft Prolaktin-senkende Mittel.

Extrakte aus Vitex agnus-castus (Keuschlamm, Mönchspfeffer) werden seit langem in der Naturheilmedizin zur Behandlung des prämenstruellen Syndroms eingesetzt. Patientinnen klagen kurz vor der Menstruation häufig über ein Spannungsgefühl in den Brüsten, was klinisch mit einem erhöhten Prolaktin-Gehalt einhergeht.

Extrakte aus Vitex agnus-castus besitzen Prolaktin-senkende Eigenschaften, die klinisch und pharmakologisch im Stand der Technik auch nachgewiesen werden konnten. Es hat im Stand der Technik nicht an Versuchen gefehlt, diejenigen Substanzen in Vitex agnus-castus, welche für eine Linderung des prämenstruellen Sydroms verantwortlich sind, zu charakterisieren oder gar zu isolieren.

So beschäftigt sich die Dissertation von Daniel Berger mit dem Titel "Vitex agnus-castus: Unbedenklichkeit und Wirksamkeit beim prämenstruellen Syndrom, Wirkprinzipien und Wirkmechanismen eines neu entwickelten Extraktes" an der Philosophisch-Naturwissenschaftlichen Fakultät der Universität Basel vom 13. Januar 1998 mit einer Vielzahl von Aspekten, die mit Vitex agnus-castus in Zusammenhang gebracht werden.

Diese Dissertation beschreibt eine Reihe von unterschiedlichen Inhaltsstoffen, welche für die Erklärung der pharmakologischen Eigenschaften der Droge in Betracht gezogen wurden.

So finden sich in Vitex agnus-castus sowohl in der Blattdroge als auch in der Fruchtdroge die Iridoidglykoside Aucubin, Agnusid und Eurostosid.

Ferner konnten die lipophilen Flavonole Casticin, Penduletin, Chrysosplenol D und der 3,6,7,4'-Tetramethylether des 6-Hydroxykämpferols aus den Früchten isoliert werden.

Ferner beschreibt der Stand der Technik, daß in den Früchten von Vitex agnus-castus Progesteron, 17α-Hydroxyprogesteron, Testosterone und Epitestosterone nachgewiesen wurden.

Darüberhinaus finden sich in den Früchten von Vitex agnus-castus noch insgesamt etwa 73 verschiedene Verbindungen, vor allem Monoterpene wie α-Pinen, Sabinen, β-Phellandren und 4-Terpineol und Sesquiterpene wie β-Caryophyllen, Alloaromadendren, Germacren B, Spathulenol und t-Cadinol.

Neben den bereits erwähnten Stoffklassen finden sich in den Früchten von Vitex agnus-castus auch erhebliche Mengen an Fettsäuren und zwar gesättigte, einfach ungesättigte und mehrfach ungesättigte Fettsäuren. So wurden beispielsweise α-Linolensäure, Oleinsäure, Stearinsäure, Palmitinsäure, Linolsäure und Adrensäure in den Früchten nachgewiesen.

Eine weitere Untersuchung des ätherischen Öls aus den Früchten von Vitex agnus-castus deckte auch das Vorkommen von Diterpenen auf. In der oben erwähnten. Dissertation finden sich Hinweise, daß aus den Früchten von Vitex agnus-castus folgende Diterpene isoliert wurden:
Rotundifuran, Vitexilacton und 6β,7β-diacetoxy-13-hydroxy-labda-8,14-dien, deren Strukturformeln im folgenden wiedergegeben sind:

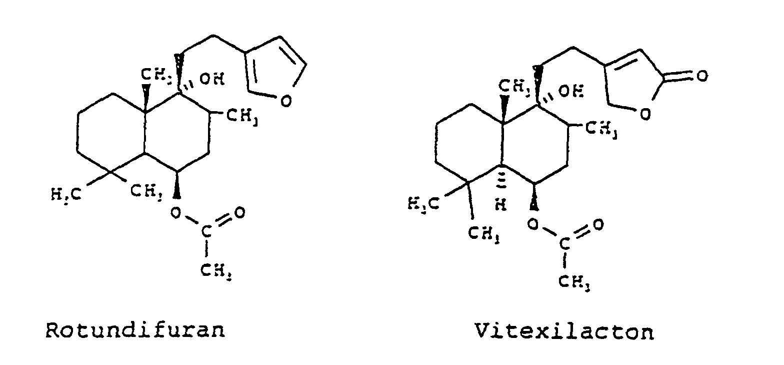

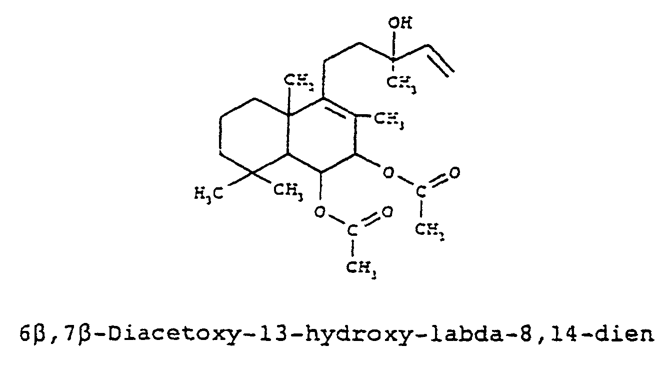

In diesem Stand der Technik wurden eine Vielzahl von Testverfahren durchgeführt, um herauszufinden, wie die Extrakte von Vitex agnus-castus wirken, und ob eine bestimmte Substanz oder eine bestimmte Substanzklasse geeignet ist, die pharmakologischen Wirkungen des Vollextraktes zu erklären.

So wurden beispielsweise Messungen an den unterschiedlichen Opioid-Rezeptoren, am Benzodiazepin-Rezeptor, an der Serotonin-Wiederaufnahmestelle, am Histamin-H₁-Rezeptor sowie am Dopamin-D₂-Rezeptor durchgeführt.

Um die Resultate der Rezeptorbindungsstudien an dem Dopamin-D₂-Rezeptor mit Fraktionen aus Vitex agnus-castus zu überprüfen und so die eigentlichen Wirksubstanzen zu finden, wurden im oben beschriebenen Stand der Technik Versuche mit den bekannten Inhaltsstoffen von Vitex agnus-castus (Reinsubstanzen) durchgeführt. Diese reinen Inhaltsstoffe waren Aucubin, Casticin, Homoorientin, Linolsäure, Luteolin-7-Glucosid, Orientin und die Diterpene Vitexin, Rotundifuran, 6β,7β-diacetoxy-13-hydroxy-labda-8,14-dien.

Die Dissertation führt jedoch explizit auf Seite 154 in Kapitel 2.3.4.5 aus, daß keine der getesteten Substanzen einen genügend tiefen IC₅₀-Wert hatte, um als Einzelsubstanz die Aktivität - also die pharmakologischen Wirkungen - des Vollextraktes oder einer lipophilen Hexanfraktion aus Vitex agnus-castus erklären zu können.

Ausgehend von diesem Stand der Technik war es Aufgabe der vorliegenden Erfindung, Reinsubstanzen aus den Früchten von Vitex agnus-castus zur Verfügung zu stellen, mit denen ein Mittel zur Behandlung des prämenstruellen Syndromes in pharmazeutischer Formulierung hergestellt werden kann.

Die Lösung dieser Aufgabe erfolgt durch ein Mittel gemäß Anspruch 1 sowie durch die neuen Substanzen gemäß Anspruch 4 und Anspruch 6.

Im Rahmen der vorliegenden Erfindung wurde überraschend gefunden, daß Verbindungen aus der Klasse der bizyklischen Diterpene eine Prolaktin-senkende Wirkung auf kultivierte Hypophysenzellen aus Ratten ausüben. Es ist sehr wahrscheinlich, daß dieser Mechanismus auf Menschen übertragbar ist.

Die wirksamen Diterpene besitzen dabei ein Grundgerüst vom Clerodan-Typ.

Insbesondere hat sich herausgestellt, daß mit Verbindungen gemäß den folgenden allgemeinen Formeln III bis IV eine Prolaktin-senkende Wirkung - bei gleichzeitig niedriger Cytotoxizität - auf kultivierte Hypophysenzellen zu erreichen ist: 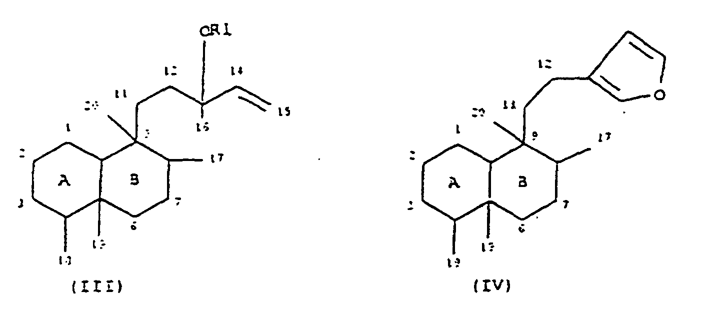 wobei R1 = H, C1 bis C3-Alkyl oder C1 bis C3-Acyl ist;
wobei die Ringe A und/oder B im Falle der allgemeinen Formeln (III) oder (IV) in Position 1, 2, 3, 4, 6, 7, oder 8 gegebenenfalls wenigstens mit einem OX-Rest substituiert sind, wobei X = H, C1 bis C3-Alkyl oder C1 bis C3-Acyl ist;
wobei gegebenenfalls wenigstens ein Kohlenstoffatom in Position 17, 18, 19 und 20 mit einem OX-Rest substituiert ist, wobei X = H, C1 bis C3-Alkyl oder C1 bis C3-Acyl ist;
wobei gegebenenfalls wenigstens eine CH3-Gruppe in Position 17, 18, 19 und 20 ersetzt ist durch eine COOH-Gruppe;
wobei gegebenenfalls wenigstens eine der Ringpositionen 1, 2, 3, 6, oder 7 eine Ketogruppe ist; und
wobei gegebenenfalls in den Ringpositionen 1, 2, 3, 6, 7, 8, 8(17) der Formel (III) wenigstens eine Doppelbindung vorliegt; und
wobei gegebenenfalls in den Ringpositionen 1, 2, 3, 4(18), 6, 7, 8, 8(17) der Formel (IV) wenigstens eine Doppelbindung vorliegt.

Ferner sind folgende Verbindungen bevorzugte Ausführungsformen der vorliegenden Erfindung: 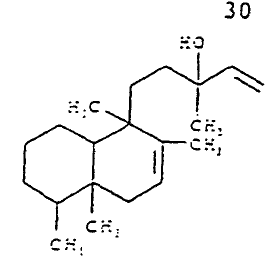 sowie
Cleroda-Y,14-dien-13-ol, wobei Y = Ringposition 1, 2, 3, 4(18), 6, 7 oder 8(17) ist; und
Cleroda-Y,Z,14-trien-13-ol, wobei Y oder Z = Ringposition 1, 3, oder 1, 4(18) oder 1, 6 oder 1, 7 oder 1, 8(17) oder Ringposition 2,4(18) oder 2, 6 oder 2, 7 oder 2, 8(17) oder Ringposition 4(18), 6 oder 4(18), 7 oder 4(18), 8(17) oder Ringposition 6, 8(17).

Derartige Verbindungen konnten aus einem ethanolischwässrigen Extrakt von Früchten aus Vitex agnus-castus durch fraktionierte lipophile Extraktion angereichert und charakterisiert werden sowie ihre Strukturen bestimmt werden.

Insbesondere die Extraktion mit sehr lipophilen Lösungsmitteln wie mittelkettigen Kohlenwasserstoffen C₅-C₁₀, insbesondere mit n-Hexan ergab eine starke Anreicherung der Prolaktin-senkenden Wirkung. Auch die Extraktion aus Früchten von Vitex agnus-castus mit überkritischem Kohlendioxid erlaubt eine starke Anreicherung des wirksamen Prinzipes, die auf die Verbindungen gemäß den allgemeinen Formeln III bis IV zurückgeführt werden konnte.

Die erfindungsgemäßen Verbindungen, deren Strukturformeln vorstehend wiedergegeben sind, zeigen alle an lactotropen Zellen aus Rattenhypophysen eine Hemmung des freigesetzten Prolactins.

In separaten Studien zur Cytotoxizität wurde festgestellt, daß sämtliche isolierten und charakterisierten erfindungsgemäßen Verbindungen eine niedrige Cytotoxizität zeigten, was sie für die pharamzeutische Formulierung besonders attraktiv macht.

Es wurde ferner festgestellt, daß die genannten Substanzen auch an den humanen rekombinanten Dopamin-D2-Rezeptor binden.

Weitere Vorteile und Merkmale der vorliegenden Erfindung ergeben sich anhand der Beschreibung von Ausführungsbeispielen sowie anhand der Zeichnung.

Es zeigt:

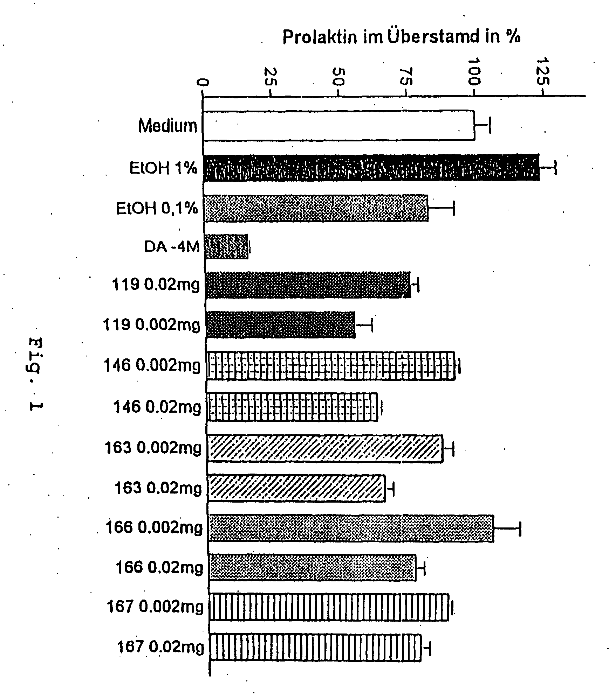

- Fig. 1: Beeinflussung der Prolaktin-Freisetzung aus kultivierten Hypophysenzellen von Ratten durch bizyklische Diterpene.

Ein ethanolischer Extrakt aus Fructus Agni casti wird in an sich bekannter Weise durch Mazeration oder Perkolation mit organischen Lösungsmitteln oder Gemischen von organischen Lösungsmitteln mit Wasser oder mit überkritischem Kohlendioxid hergestellt.

Vorzugsweise benutzt man dazu Mischungen von Ethanol mit Wasser im Verhältnis 50:50 bis 90:10 bei Temperaturen von 20 bis 60 Grad Celsius.

Der so erhaltene Extrakt wird zwischen zwei unterschiedlich polaren nicht mischbaren Phasen verteilt. Dazu setzt man Alkane, Halogenkohlenwasserstoffe, Ketone, Ester als lipophile Phase und Alkohole und Wasser als hydrophile Phase ein. Vorzugsweise verwendet man gleiche Volumina von C5 bis C7-Alkanen und Ethanol / Wasser Gemische im Verhältnis 1:2 bis 1:10)

Die lipophile Phase enthält die Prolaktin-senkende Aktivität und kann mittels bekannter Verfahren wie zum Beispiel Hochdruckflüssigkeitschromatographie und Präparativer Schichtchromatographie in an sich bekannter Weise weiter aufgereinigt werden.

Aus 1 kg gemahlener Früchte von Vitex Agnus castus stellt man durch Perkolation mit 10 1 Ethanol/Wasser 6/4 (v/v) einen Extrakt her. Ein daraus hergestellter Dickeextrakt mit einem Trockenrückstand von 1,75 g wird zwischen 375 ml 15% EtOH und 375 ml n-Hexan im Scheidetrichter verteilt, die n-Hexan-Phase wird abgenommen und die wässrige Phase wird erneut mit n-Hexan ausgeschüttelt.

Die vereinigten Hexan-Phasen ergeben nach Aufkonzentration im Vakuum einen Rückstand von 300 mg.

Der so erhaltene Rückstand wird mittels Hochdruckflüssigkeitschromatographie weiter aufgetrennt. Dazu verwendet man eine Säule mit den Dimensionen 21,4 x 300 mm, mit C-18 Material der Korngröße 8 µm als stationäre Phase. Die Chromatographie erfolgt bei einem Fluß von 10ml /min eines Gemisches von Acetonitril/Wasser 60/40 als Laufmittel. Nach Aufgabe der Probe wird der Anteil an Acetonitril innerhalb von 60 Minuten linear auf 100 % erhöht.

In dem Volumen zwischen 350 und 450 ml eluieren die gesamten Diterpene. Aus 300 mg Hexanphase erhält man ca. 38 mg Diterpen-Gemisch. Die Diterpene werden zweckmässicrerweise fraktioniert aufgefangen.

Die weitere Aufreinigung des so erhaltenen Diterpengemischs erfolgt mittels Präparativer Schichtchromatographie an Kieselgelschichten mit 1mm Schichtdicke mit unterschiedlichen Fliessmitteln wie weiter unten für die einzelnen Substanzen beschrieben sind. Die Detektion erfolgt mit Anisaldehyd-Reagenz ( DAB 10, 1997). Die Zonen der reinen Diterpene auf der Dünnschichtplatte werden mit Chloroform/Methanol eluiert und mittels gekoppelter Gaschromatographie-Massenspektrometrie analysiert.

### Darstellung von 98-146 (in Fig. 1 mit "146" bezeichnet) (Vergleichsbeispiel):

6β-Acetoxy-9α-hydroxy-15,16-epoxy-13(16),14-labdadien (Rotundifuran) 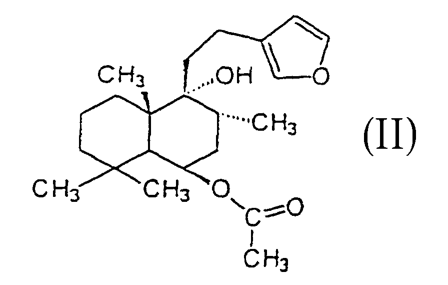 Fließmittel: Chloroform / Methanol 95/5
Rf-Wert: 0,75
Charakteristische Fragmente der underivatisierten Substanz im GC-M5,:
m/z=362 [M]+ , 344, 302, 287, 284, 207, 150, 135, 95, 81.

### Darstellung von 98-119 (in Fig 1. Mit "119" bezeichnet):

Cleroda-y,14,-dien-13-ol
Fließmittel: Chloroform / Methanol 95/5
Rf-Wert: 0,63
Charakteristische Fragmente der underivatisierten Substanz im GC-MS,:
m/z=290 [M]+ , 272, 257, 243, 229, 191, 189,

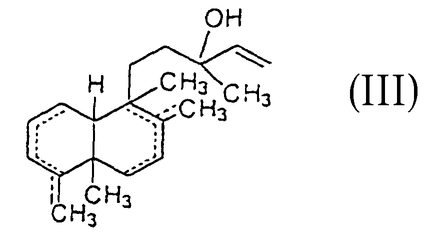

### Darstellung von 98-153 (in Fig. 1 nicht gezeigt) (Vergleichsbeispiel):

x,13-Dihydroxy-14-labden Fließmittel: Chloroform / Methanol 95/5
Rf-Wert: 0,37
Charakteristische Fragmente der underivatisierten Substanz im GC-MS, :
m/z=290 [M-H2O]+ , 275, 272, 257, 191, 177

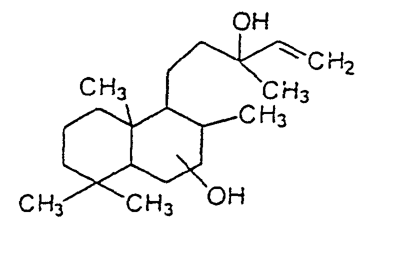

### Darstellung von 98-152 (in Fig. 1 nicht gezeigt) (Vergleichsbeispiel):

6β,7β-Diacetoxy-13-hydroxy-labda-8,14-dien
Fließmittel: Chloroform / Methanol 99/1
Fließstrecke 16cm, dreimal entwickelt
Rf-Wert: 0,5
Charakteristische Fragmente der underivatisierten Substanz im GC-MS,:
m/z=346 [M-60]⁺ , 307, 304, 286, 247, 205, 187, 177, 135

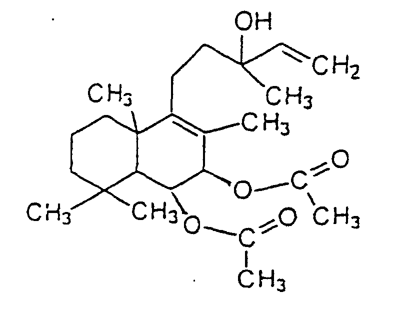

### Darstellung von 98-166 (in Fig. 1 mit "166" bezeichnet) (Vergleichsbeispiel):

x-Hydroxy-y-keto-15, 16-epoxy-13 (16),14-labdadien Fließmittel: Chloroform / n-Hexan 90/10
dreimal entwickeln, Laufstrecke 16cm
Rf-Wert: 0,74
Charakteristische Fragmente der underivatisierten Substanz im GC-MS,:
m/z=318 [M]⁺ , 300, 285, 193, 166, 95, 81

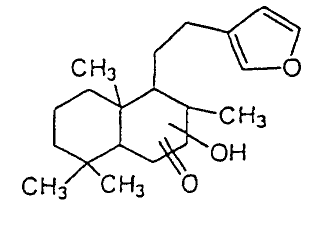

### Darstellung von 98-167 (in Fig. 1 mit "167" bezeichnet) (Vergleichsbeispiel):

x-Acetoxy-13-hydroxy-labda-y,14-dien
Fließmittel: Chloroform / n-Hexan 90/10
dreimal entwickeln, Laufstrecke 16cm
Rf-Wert: 0,55
Charakteristische Fragmente der underivatisierten Substanz im GC-MS,:
m/z=330 [M-H2O]⁺ 288, 270, 255, 249, 189, 132, 119, 71

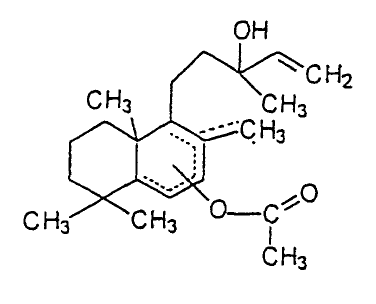

### Beeinflussung der Prolaktinfreisetzung:

Die Bestimmung der Freisetzung von Prolaktin aus kultivierten Hypophysenzellen von männlichen Ratten wurde durchgeführt wie bei Jarry et. al., Experimental and Clinical Endocrinology, Vol.102, (1994) 448 - 454, beschrieben. Die Diterpene wurden in ethanolischer Lösung zu den Zellkulturen gegeben. Die entsprechenden Ethanolkonzentrationen und Dopamin wurden als Kontrollen mitgeführt.

Der Mittelwert der gemessenen Prolaktinkonzentration von Überständen von Zellen die in unsupplementiertem Medium inkubiert wurden, ist gleich 100 Prozent gesetzt. Die Diterpene erniedrigen signifikant die Freisetzung von Prolaktin. Die Ergebnisse sind in Fig 1 dargestellt. Sie zeigt die Senkung der Prolaktinfreisetzung aus kultivierten Hypophysenzellen von Ratten durch bizyklische Diterpene. Als Kontrolle wurden Medium, Medium plus Ethanol und 10⁻⁴ molar Dopamin (=DA-4M) mitgeführt. Die Konzentration ist in mg Diterpen pro ml Medium angegeben.

## Patentansprüche

1. Prolaktin-senkendes Mittel, enthaltend wenigstens ein bicyclisches Diterpen vom Clerodantyp gemäß wenigstens einer der allgemeinen Formeln (III) oder (IV): 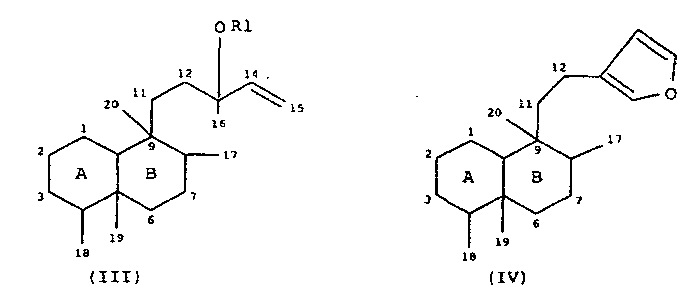 wobei R1 = H, C₁ bis C₃-Alkyl oder C₁ bis C₃-Acyl ist;
wobei die Ringe A und/oder B im Falle der allgemeinen Formeln (III) oder (IV) in Position 1, 2, 3, 4, 6, 7, oder 8 gegebenenfalls wenigstens mit einem OX-Rest substituiert sind, wobei X = H, C₁ bis C₃-Alkyl oder C₁ bis C₃-Acyl ist;
wobei gegebenenfalls wenigstens ein Kohlenstoffatom in Position 17, 18, 19 und 20 mit einem OX-Rest substituiert ist, wobei X = H, C₁ bis C₃-Alkyl oder C₁ bis C₃-Acyl ist;
wobei gegebenenfalls wenigstens eine CH₃-Gruppe in Position 17, 18, 19 und 20 ersetzt ist durch eine COOH-Gruppe;
wobei gegebenenfalls wenigstens eine der Ringpositionen 1, 2, 3, 6, oder 7 eine Ketogruppe ist; und
wobei gegebenenfalls in den Ringpositionen 1, 2, 3, 6, 7, 8, 8(17) der Formel (III) wenigstens eine Doppelbindung vorliegt; und
wobei gegebenenfalls in den Ringpositionen 1, 2, 3, 4(18), 6, 7, 8, 8(17) der Formel (IV) wenigstens eine Doppelbindung vorliegt;
ausgenommen folgende Verbindungen: (+) Hardwickiische Säure, Crolechinsäure und Hautriwaische Säure.

2. Prolaktin-senkendes Mittel nach Anspruch 1, **dadurch gekennzeichnet, daß** es eine Verbindung mit folgender Formel enthält:

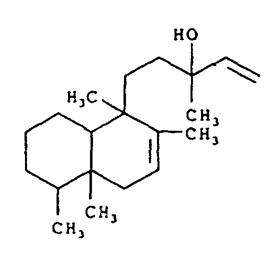

3. Verwendung eines Prolaktin-senkenden Mittels gemäß einem der Ansprüche 1 oder 2 zur Herstellung eines Arzneimittels zur Behandlung des prämenstruellen Syndroms, Mastodynie, Zyklusanomalien, insbesondere Oligo- bis Amenorrhoe.

4. Cleroda-Y, 14-dien-13-ol, wobei Y = Ringposition 1, 2, 3, 4(18), 6, 7 oder 8(17) ist.

5. Verwendung von Cleroda-Y,14-dien-13-ol, wobei Y = Ringposition 1, 2, 3, 4(18), 6, 7 oder 8(17) ist, zur Herstellung eines Arzneimittels zur Prolaktin-Senkung oder zur Behandlung des prämenstruellen Syndroms, Mastodynie, Zyklusanomalien, insbesondere Oligo- bis Amenorrhoe.

6. Cleroda-Y,Z,14-trien-13-ol, wobei Y oder Z = Ringposition 1, 3, oder 1, 4(18) oder 1, 6 oder 1, 7 oder 1, 8(17) oder Ringposition 2,4(18) oder 2, 6 oder 2, 7 oder 2, 8(17) oder Ringposition 4(18), 6 oder 4(18), 7 oder 4(18), 8(17) oder Ringposition 6, 8(17).

7. Verwendung von Cleroda-Y,Z,14-trien-13-ol, wobei Y oder Z = Ringposition 1, 3, oder 1, 4(18) oder 1, 6 oder 1, 7 oder 1, 8(17) oder Ringposition 2,4(18) oder 2, 6 oder 2, 7 oder 2, 8(17) oder Ringposition 4(18), 6 oder 4(18), 7 oder 4(18), 8(17) oder Ringposition 6, 8(17), zur Herstellung eines Arzneimittels zur Prolaktin-Senkung oder zur Behandlung des prämenstruellen Syndroms, Mastodynie, Zyklusanomalien, insbesondere Oligo- bis Amenorrhoe.

## Claims

1. A prolactin lowering drug containing at least one bicyclic diterpene of the derodane type in accordance with at least one of general formulae (III) or (IV): 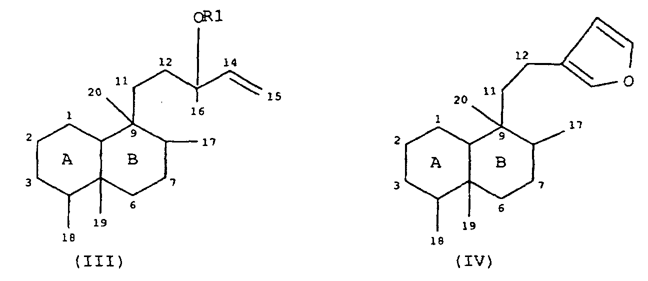 with R1 = H, C₁ to C₃ alkyl or C₁ to C₃ acyl;
wherein the rings A and/or B in the case of general formulae (III) or (IV) are optionally substituted in position 1, 2, 3, 4, 6, 7, or 8 with at least one OX radical, with X = H, C₁ to C₃ alkyl or C₁ to C₃ acyl;
wherein optionally at least one carbon atom in position 17, 18, 19 and 20 is substituted with an OX radical, with X = H, C₁ to C₃ alkyl or C₁ to C₃ acyl;
wherein optionally at least one CH₃ group in position 17, 18, 19 and 20 is replaced by one COOH group;
wherein optionally at least one of ring positions 1, 2, 3, 6, or 7 is a keto group; and
wherein optionally at least one double bond is present in ring positions 1, 2, 3, 6, 7, 8, 8(17) of formula (III); and
wherein optionally at least one double bond is present in ring positions 1, 2, 3, 4(18), 6, 7, 8, 8(17) of formula (IV);
with the exception of the following compounds: (+) hardwickiic acid, crolechinic acid and hautriwaic acid.

2. A prolactin lowering drug according to claim 1, **characterized by** containing a compound having the following formula:

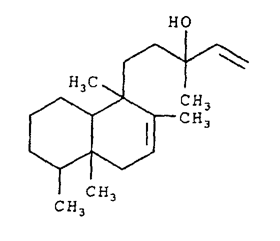

3. Use of a prolactin lowering drug, in particular one in accordance with any one of claims 1 or 2, for preparing a drug for treatment of the premenstrual syndrome, mastodynia, disorders of the menstrual cycle, in particular oligo- to amenorrhea.

4. Cleroda-Y,14-dien-13-ol, wherein Y = ring position 1, 2, 3, 4(18), 6, 7 or 8(17).

5. Use of cleroda-Y,14-dien-13-ol, wherein Y = ring position 1, 2, 3, 4(18), 6, 7 or 8(17), for preparing a prolactin lowering drug or for treatment of the premenstrual syndrome, mastodynia, disorders of the menstrual cycle, in particular oligo- to amenorrhea.

6. Cleroda-Y,Z,14-trien-13-ol, wherein Y or Z = ring position 1, 3, or 1, 4(18) or 1, 6 or 1, 7 or 1, 8(17) or ring position 2, 4(18) or 2, 6 or 2, 7 or 2, 8(17) or ring position 4(18), 6 or 4(18), 7 or 4(18), 8(17) or ring position 6, 8(17).

7. Use of cleroda-Y,Z,14-trien-13-ol, wherein Y or Z = ring position 1, 3, or 1, 4(18) or 1, 6 or 1, 7 or 1, 8(17) or ring position 2, 4(18) or 2, 6 or 2, 7 or 2, 8(17) or ring position 4(18), 6 or 4(18), 7 or 4(18), 8(17) or ring position 6, 8(17), for preparing a prolactin lowering drug or for treatment of the premenstrual syndrome, mastodynia, disorders of the menstrual cycle, in particular oligo- to amenorrhea.

## Revendications

1. Agent abaissant la teneur en prolactine, contenant au moins un diterpène bicyclique de type clérodane selon au moins l'une des formules générales (III) ou (IV) : 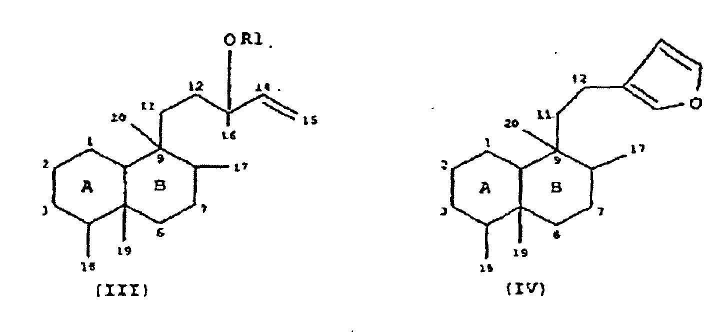 dans laquelle R1 = H, alkyle en C₁ à C₃ ou acyle en C₁ à C₃,
dans laquelle les cycles A et/ou B dans le cas des formules générales (III) ou (IV) sont éventuellement substitués en position 1, 2, 3, 4, 6, 7 ou 8, avec au moins un radical OX, X = H, alkyle en C₁ à C₃ ou acyle en C₁ à C₃,
dans laquelle éventuellement au moins un atome de carbone est substitué en position 17, 18, 19 et 20 avec un radical OX, X = H, alkyle en C₁ à C₃, ou acyle en C₁ à C₃,
dans laquelle éventuellement au moins un groupe CH₃- est remplacé en position 17, 18, 19 et 20, par un groupe COOH ;
dans laquelle éventuellement au moins une des positions cycliques 1, 2, 3, 6 ou 7 est un groupe cétone ; et
dans laquelle éventuellement dans les positions cycliques 1, 2, 3, 6, 7, 8, 8(17) de la formule (III) se trouve au moins une double liaison ; et
dans laquelle éventuellement dans les positions cycliques 1, 2, 3, 4(18), 6, 7, 8, 8(17) de la formule (IV) se trouve au moins une double liaison ;
excepté les composés suivants :
(+) acide de Hardwicki, acide croléchinique et l'acide hautriwaïque.

2. Agent abaissant la teneur en prolactine selon la revendication 1, **caractérisé en ce qu'**il contient un composé de formule suivante :

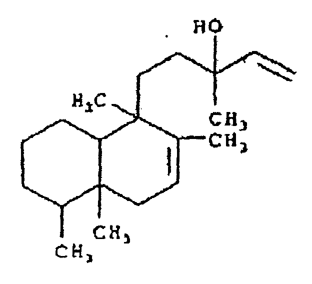

3. Utilisation d'un agent abaissant la teneur en prolactine selon l'une quelconque des revendications 1 ou 2, pour produire un médicament pour le traitement du syndrome prémenstruel, de la mastodynie, des anomalies du cycle, en particulier des oligoménorrhées aux aménorrhées.

4. Composé cléroda-Y,14-diène-13-ol, dans lequel Y est la position cyclique 1, 2, 3, 4(18), 6, 7 ou 8(17).

5. Utilisation du composé cléroda-Y,14-diène-13-ol, dans lequel Y est la position cyclique 1, 2, 3, 4(18), 6, 7 ou 8(17) pour produire un médicament destiné à réduire la teneur en prolactine ou pour le traitement du syndrome prémenstruel, de la mastodynie, des anomalies du cycle, en particulier des oligoménorrhées aux aménorrhées.

6. Composé cléroda-Y,Z, 14-triène-13-ol, dans lequel Y ou Z est la position cyclique 1, 3 ou 1, 4(18) ou 1, 6 ou 1, 7 ou 1, 8(17) ou la position cyclique 2, 4(18) ou 2, 6 ou 2, 7 ou 2, 8(17) ou la position cyclique 4(18), 6 ou 4(18), 7 ou 4(18), 8(17) ou la position cyclique 6, 8(17).

7. Utilisation du composé cléroda-Y,Z,14-triène-13-ol, dans lequel Y ou Z est la position cyclique 1, 3 ou 1, 4(18), ou 1, 6 ou 1, 7 ou 1, 8(17) ou la position cyclique 2, 4(18) ou 2, 6 ou 2,7 ou 2,8(17) ou la position cyclique 4(18), 6 ou 4(18), 7 ou 4(18), 8(17) ou la position cyclique 6, 8(17) pour produire un médicament destiné à réduire la teneur en prolactine ou pour le traitement du syndrome prémenstruel, de la mastodynie, des anomalies du cycle, en particulier des oligoménorrhées aux aménorrhées.
